# EUROPEAN PATENT APPLICATION

(11) **EP 1 241 264 A1**
(43) Date of publication of application: **18.09.2002**
(21) Application number: 02005019.1
(22) Date of filing: 28.11.1995
(51) Int. Cl.: C12P 21/08, C07K 2/00, C12N 15/02, A61K 39/00, A61K 39/395

(54) **Monoclonal antibodies to colon cancer antigen**

(30) Priority: 02.12.1994 US 349489; 07.06.1995 US 485786
(62) Divisional of application: 95941489.7
(71) Applicant: CHIRON CORPORATION, Emeryville California 94608-2916 (US)
(72) Inventor: Ring, David B., Palo Alto, CA 94301 (US)
(74) Representative: Duckworth, Timothy John

(57) **Abstract**

A monoclonal antibody which is obtainable from the hybridoma deposited with the American Type Culture Collection having Accession No. HB 11751, antigen bound by the monoclonal antibody and monoclonal antibodies that bind to the antigen. Use of such antibodies and antigens in the manufacture of medicaments for inducing an immune response or for diagnosing or treating cancer.

## Description

### Background of the Invention

The invention relates to methods of inducing or enhancing an immune response in a patient using bispecific antibodies in which one arm of the antibody recognizes and binds to FcγRIII, also called CD16. Bispecific antibodies useful in the method of the invention include bispecific antibodies to FcγRIII and a cancer antigen such as, for example, the bispecific antibody 2B1 which recognizes and binds c-*erb*B-2 antigen. In addition, the invention also relates to induction of an immune response in a patient using bispecific antibodies to FcγRIII and a viral antigen, or other antigen.

Use of bispecific antibodies for induction of an immune response employs the principles of the biological properties of antibodies derived from the structure of their polypeptide components, the heavy and light chains. The heavy chain is about twice the size of the light chain, and a stoichiometry for each antibody is H₂-L₂. The heavy and light chains are held together by noncovalent forces and by covalent interchain disulfide bridges. The heavy chain is made up of 4 folded globular domains, and the light chain of 2. Each globular domain includes about 100 to 110 amino acids. In the heavy chain the variable region V_{H} forms the amino terminal end and constant regions C_{H1}, C_{H2}, and C_{H3} form the remainder. The light chain consists of the variable region V_{L} and the constant region C_{L1}. A V_{H} region aligned with the corresponding V_{L} region forms the antigen binding region or antigen-recognition site of the antibody molecule.

The extreme sequence diversity of the variable regions of the antibody molecules generated during differentiation of antibody producing cells allows an organism to produce antibodies capable of recognizing a wide variety of antigens including proteins, simple and complex carbohydrates, simple intermediary metabolites, sugars, lipids, hormones, phospholipids, and nucleic acids. The variable regions of the heavy and light chains each consist of 4 relatively invariant stretches called framework regions (FRs) of 15-30 amino acids separated by 3 shorter regions of extreme variability, called hypervariable regions or complementarity determining regions (CDRs) of 5-12 amino acids. The sequences of these CDRs are the primary determinants of antigen specificity. The CDRs within each chain are designated CDR1, CDR2, and CDR3, beginning at the N-terminus, as described in Parslow *et al*, Basic and Clinical Immunology, (8th Ed. Appleton & Lange, 1994) chapter 6.

Antibodies have two binding sites, both of which normally recognize the same epitope. However, interest in bispecific antibodies having binding specificity for two different epitopes has recently developed. Bispecific antibodies may be formed by chemically conjugating two different antibodies. Alternatively, bispecific antibodies may be isolated from a hybrid hybridoma, a cell fusion of two monoclonal antibody-producing cells, as shown, for example, in Ring *et al,* Breast Epithelial Antigens: Molecular Biology to Clinical Applications, Ceriani, ed. (Plenum Press, NY, 1991) pp. 91-104, and U.S. 4,714,681, the disclosures of which are herein incorporated by reference.

There are at least three Fc receptors, including FcγRI, FcγRII, and FcγRIII which has two isoforms A and B. The FcγRIIIA isoform, also called CD16, is a 50-70 kD cell surface molecule capable of binding the Fc portion of an IgG with an affinity of 2x10⁻⁶M, and is found on natural killer (NK) cells, neutrophils, eosinophils, γ/δ lymphocytes, macrophages, and some monocytes. Perussia *et al, J. of Immunology*, (1991) 132:1410-1415. Throughout this discussion, FcγRIII should be understood as including the FcγRIIIA isoform only. FcγRIII is a low affinity receptor and more efficiently binds aggregated IgG than monomeric IgG. NK cells use their FcγRIII surface bound molecule to bind Fc portions of antibodies, themselves bound to antigens, for example, on the surface of target cells. Binding of the Fc portions of antibodies to the FcγRIII molecule on NK cells, activates the NK cells to lyse the target cell. NK cells also express homodimers of the CD3 ζ chain or a homodimeric protein called γ. Both homodimers are thought to be involved in signal transduction initiated by IgG binding to FcγRIII.

Snider *et al, J. Immunol.* (1987) 139:1609-16 first described using bispecific antibodies to target an antigen to an Fc receptor on an antigen-presenting cell in an *in vitro* system. This study used antibody heteroaggregates containing an antibody against a protein antigen covalently crosslinked to an antibody against a target structure on the surface of the antigen presenting cells. Antigen presentation was assessed by measurement of lymphokine released by antigen specific T cell hybridomas. Enhanced presentation occurred when antigen was targeted to MHC class I and class II molecules, surface immunoglobin, or Fcg receptors on the surface of a murine B cell lymphoma/hybridoma. The ability of each crosslinked antibody to enhance presentation paralleled the total amount of each that bound to the surface of the antigen presenting cells. This research also found that the presentation of one antigen using crosslinked antibody did not result in enhanced presentation of a second, bystander antigen.

Anti-tumor effects of a bispecific antibody with affinity for FcγRIII and CA19-9 cancer antigen have been shown in mice as described in Palazzo *et al, Cancer Research* (1992) 52:5713-5719. In this report, it was first shown that bispecific antibody CL158 mediated lysis of adenocarcinoma-like cells (SW948 tumor cells) growing in a monolayer in the presence of IL-2 activated whole blood. The lysis of the tumor cells monitored by^{In} release occurred after 4 hours. Next, when SW948 cells were grown *in vitro* as multicellular human tumor spheroids, it was shown that incubation with interleukin-2-activated lymphocytes (LAK cells) and CL158 which recognizes FcγRIII and CA19-9 cancer antigen, caused widespread structural lysis of the tumor cells. Lysis of the tumor in spheroids occurred after 24 hours.

Palazzo *et al* also described a scid mouse xenograft animal model bearing subcutaneous tumors employed to test the potential *in vivo* efficacy of the bispecific antibody. They showed that bispecific murine monoclonal antibodies which target tumor and FcγRIII and tumor antigen can promote relevant tumor lysis in mice by large granular lymphocytes. In this study the successful lysis occurred upon treatment of the mice with LAK cells, IL-2 and the bispecific antibody CL158. It was acknowledged that cytotoxicity assays that measure radioactivity release address tumor lysis capability but do not address the ability of cytotoxic effector cells to infiltrate and mediate destruction of solid tumors. The narrow success of the research demonstrated in this work is dependent on the close physical association of the LAK cells to the tumor cells.

Exploitation of the ability of monoclonal antibodies to target tumors for lysis by cellular effector mechanisms has been shown in mice as described in Weiner *et al, Cancer Res.* (1993) 53:94-100. See also Hsieh-Ma *et al, Cancer Res*. (1992) 52:6832-39. In this study, the effects of 2B1, a bispecific monoclonal antibody with specificity for the extracellular domain of the c-*erb*B-2 oncogene product and the human Fcγ receptor, FcγRIII, was examined. Administration of 2B1 with IL-2 activated LAK or PBL cells increased the median survivals in mice expressing c-*erb*B-2 antigen. These effects were preserved when the bispecific antibody and human effector cells were administered in human serum, which inhibits Fc domain-mediated effects, while the antitumor properties of 520C9, the parent anti-c-*erb*B-2 antibody of 2B1, were completely abrogated by serum or administration of the F(ab')2 fragment. The success of this study remained dependent on the co-administration of IL-2 activated cells, and suggested that human tumors implanted in mice can be successfully targeted by 2B1-based therapy provided that appropriate human effector cells are co-administered.

Snider *et al, J. Exp. Med.* (1990) 171:1957-63, first reported that bispecific antibody targeting of antigen to an Fc receptor on an antigen presenting cell (APC) in mice resulted in enhanced immunogenicity of the antigen in the mice. Bispecific antibodies were prepared by chemically crosslinking an antibody with specificity to hen egg lysozyme (HEL) to various other antibodies, including an antibody to FcγRII, and anti-I-A, and anti-IgD, each specific for a particular APC cell surface component. This study showed that heterocrosslinked bispecific antibodies, when administered once with nanogram amounts of antigen, in the absence of adjuvant, induced high titers of antibody in mice, and primed mice for a secondary IgG antibody response when rechallenged with soluble antigen.

Thus far in research studying the efficacy of bispecific antibodies, localized tumor cell lysis has been observed in cellular and murine *in vivo* studies where the effector cells have been in close proximity to the tumor cells upon administration of the bispecific antibody.

It would be clearly advantageous to determine the efficacy of administration of bispecific antibodies in humans, and to develop a method of promoting a beneficial immune response in humans having cancer, a viral infection, or other disease known to evade the immune system.

### Summary of the Invention

The invention relates to a method of inducing an immune response in a patient having the step of administering a bispecific antibody to the patient, said bispecific antibody having a first binding site capable of recognizing and binding a first antigen wherein the first antigen is FcγRIII and also having a second binding site capable of recognizing and binding a second antigen. The first binding site may be a binding site derived from the monoclonal antibody produced from the 3G8 hybridoma.

The second antigen recognized by the bispecific antibody may be present in the patient. The second antigen may be a self antigen and the self antigen may be a cancer antigen. The cancer antigen may be any of a 145 kD tumor protein, a 42 kD tumor glycoprotein, or a glycolipid. The second binding site capable of recognizing and binding the cancer antigen may be a binding site derived from a monoclonal antibody produced by any of the following hybridomas: 42H8 (HB11830), 35E6 (HB11769) or 36H3 (HB 11768).

The cancer antigen may also be a squamous cell carcinoma antigen, a 300,000 Dalton glycoprotein breast cancer antigen, or a 195 kD tumor-associated antigen. The cancer antigen may also be a tumor-associated ganglioside.

The cancer antigen may be one of the tumor-associated gangliosides GD3, GD2, GM3, GM1, G2, 6C, Le^{a}, or Le^{x}.

The second antigen of the invention may be a viral antigen expressed by a virus from the group of viruses: HIV, HAV, HBV, HCV, HPV, HSV, CMV, EBV and Influenza viruses.

The viral antigen may be an HIV antigen, specifically an HIV glycoprotein, more specifically HIV glycoprotein gp120, even more specifically the V3-PND region of HIV glycoprotein antigen gp120.

The viral antigen may be an HAV antigen, and may bind anti-HAV monoclonal antibodies K3-4C8 and B5-B3.

The viral antigen may be an HBV antigen, specifically an HBV antigen located in the pre-S1, pre-S2, or S region-encoded domains of HBV proteins.

The viral antigen may be an HCV antigen, specifically either E1 or E2 HCV antigen.

The viral antigen may be HPV antigen, specifically an HPV type II antigen.

The viral antigen may be an HSV antigen, specifically an antigen to the envelope glycoprotein of HSV, and more specifically antigen to antibody clone numbers A19080023A, A19090023A, A19100023A, A19170023A, and A19180023A.

The viral antigen may be a CMV antigen, specifically an immediate early nonstructural antigen, immediate early, early or late antigen to CMV, or an antigen to the major CMV envelope glycoprotein, gB (gp UL 55). More specifically, the antigen may bind antibodies identified clone numbers A28020069P and A28030069P, and antibodies to the major CMV envelope glycoprotein, gB (gp UL 55).

The viral antigen may be an EBV antigen, specifically the EBV latent membrane protein, the Epstein-Barr virus nuclear antigen-2, or the viral capsid antigen (VCA).

The viral antigen may be an Influenza virus antigen, specifically an antigen to Influenza A or Influenza B virus, more specifically the antigen may bind Influenza A virus antibody clone A60010044P or Influenza B virus antibody clone number A60020044A.

The second antigen may be a fungal antigen, more specifically the second antigen may be fungal antigen polysaccharide of C. neoformans.

The second antigen may be a parasitic antigen, more specifically, an antigen to malaria parasite, even more specifically the malarial peptide PfEMP3.

The second antigen may be a toxin, more specifically tetanus toxin, and even more specifically the heavy C chain fragment of tetanus toxin.

The second antigen may also not be present in the patient upon first administration of the bispecific antibody.

### Detailed Description of the Invention

It has now been found that administration of bispecific antibodies which recognize and bind FcγRIII and a second antigen can promote an immune response in humans to the second antigen. The immune response includes the formation of antibodies to the second antigen. The second antigen can be present on the surface of cells and can be a self antigen, including cancer antigens.

The term "immune response" as used herein refers to both humoral and cell-mediated immune responses. The humoral branch of the immune system involves interaction of B cells with antigen and their subsequent proliferation and differentiation into antibody-secreting plasma cells. The antibody functions as the effector molecule of the humoral response by binding to antigen and neutralizing it or facilitating its elimination. Antibodies can cross-link the antigen, forming clusters that are more readily digested by phagocytic cells. Binding of antibody to antigen can also activate the complement system resulting in lysis of the cell to which the antibody binds including foreign organisms. Antibody can also neutralize toxins or viral particles by coating them and preventing binding to host cells.

The cell-mediated branch of the immune response occurs when effector T cells are generated in response to antigen. Both T helper cells (T_{H}) and cytotoxic T lymphocytes (CTLs) serve as effector cells in cell-mediated immune reactions. Lymphokines secreted by T_{H} cells can activate various phagocytic cells to phagocytose and kill microorganisms. Activated cytotoxic T lymphocytes participate in cell-mediated immune reactions by killing altered self-cells, virally infected cells and tumor cells. The antitumor activity of natural killer (NK) cells differs from that of CTLs because NK cells do not express antigen-specific T-cell receptors or CD3, because antigen recognition by NK cells is not MHC restricted, because the NK response generates no immunological memory, and because NK cells have been shown to be specially responsive to tumor cells and virally infected cells. NK mediated cytotoxicity may involve a process similar to CTL-mediated cytotoxicity.

A branch of cell-mediated immune response is called antibody-dependent cell-mediated cytotoxicity (ADCC). A variety of cells exhibit ADCC, including NK cells, macrophages, monocytes, neutrophils, and eosinophils. These cells express receptors for the Fc portions of antibodies such as FcγRIII. ADCC killing of cells infected with virus has been demonstrated by co-administration of anti-viral antibodies and macrophages. Target cell killing by ADCC involves a number of different mechanisms. Upon binding to target cells, macrophages become more metabolically active and phagocytose the target cell. Other cells such as activated monocytes and NK cells may secrete tumor necrosis factor and perform, a molecule which damages the integrity of the membrane of the target cell.

The term "patient" as used herein refers to a mammal capable of expressing FcγRIII on the surface of cells, the mammal being presently afflicted with or potentially afflicted with any number of diseases treatable by the method of the invention. Such diseases include, for example, cancer, viral infection, bacterial infection, parasitic infection, fungal infection, or toxin poisoning.

The term "administration" as used herein refers to the introduction into a patient of bispecific antibodies useful in the method of the invention, and more specifically means administration of an amount effective for induction of an immune response in the patient, the administration given either in a single dose or as part of a series of doses. The effective amount will vary depending upon the health and physical condition of the individual to be treated, the capacity of the individual's immune system to form antibody and T cell responses, the degree of protection desired, the formulation and other relevant factors known to those skilled in the art. The effective amount will be in a range that can be determined through routine trials by a person skilled in the art.

A pharmaceutically acceptable carrier for administration of a bispecific antibody refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art. Exemplary pharmaceutically acceptable salts include mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., NJ 1991). Therapeutic compositions may contain pharmaceutically acceptable vehicles, such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared.

The term "coadministration" as used herein refers to administration of a compound of the invention in combination with a second therapeutic agent. Coadministration may be simultaneous, for example by administering a mixture of the therapeutic agents including bispecific antibodies, or may be accomplished by administration of the agents separately within a short time period.

The term "bispecific antibody" as used herein refers to an antibody that has binding sites for two different antigens within a single antibody molecule. It will be appreciated by those skilled in the art that other molecules in addition to the canonical antibody structure may be constructed with two binding specificities. Such structures are discussed herein and are intended to be useful within the scope of the present invention. It will further be appreciated that antigen binding by bispecific antibodies may be simultaneous or sequential. Triomas and hybrid hybridomas are two examples of cell lines that can secrete bispecific antibodies. Examples of bispecific antibodies produced by a hybrid hybridoma or trioma are disclosed in U.S. 4,474,893, and Shi *et al, J*. *Immun. Meth.* (1991) 141:165-75, the disclosures of which are both herein incorporated by reference. Bispecific antibodies useful in the method of the invention may be made by the following method as described in Ring *et al*, Breast Epithelial Antigens: Molecular Biology to Clinical Applications, Ceriani ed. (Plenum Press, NY 1991) pg. 91-104. Bispecific antibody conjugates have been constructed by chemical means as described in Staerz *et al, Nature* (1985) 314:628-631 and Perez *et al, Nature* (1985) 316:354-356.

The term "antigen" as used herein refers to a molecule which is capable of binding to an appropriate T cell antigen receptor or an antibody. Antigens may comprise proteins, protein fragments, simple and complex carbohydrates, simple intermediary metabolites, sugars, lipids, hormones, phospholipids, and nucleic acids. An epitope is the portion of an antigen which is capable of being recognized and bound by an antibody or T cell antigen receptor. It will be appreciated that a given antigen may have more than one epitope.

The term "FcγRIII" as used herein refers to a cell surface protein, also known in the literature as CD 16. FcγRIII as used herein refers to the A isoform present on cell surfaces and is distinguished from the B isoform which is shed by cells into the circulation. FcγRIII has a molecular weight in the range of 50-70 kD. FcγRIII binds aggregated IgG more efficiently than monomeric IgG. FcγRIII is a low affinity receptor for the Fc portion of IgG, binding IgG with an affinity of 2 X 10⁻⁶ M. FcγRIII is known to be expressed on NK cells, γ/δ T lymphocytes, macrophages, eosinophils and neutrophils. NK cells can employ FcγRIII to attach to target cells by binding to the Fc region of antibodies, themselves bound to antigen on the surface of target cells. Binding of the Fc portions of antibodies to the FcγRIII molecule on NK cells, activates the NK cells to lyse the target cells.

The term "antibody" as used herein also includes antibodies which retain the antigen binding function of the parent antibody. The parent antibody is the antibody originally generated in an immune response to a specific antigen. Antibodies can also include, for example, polyclonal, monoclonal, chimeric, humanized, hybrid (also called bispecific), and altered antibodies.

The term "binding site derived from a monoclonal antibody" as used herein means a binding site in a second antibody or antibody fragment having the same or homologous CDRs as the monoclonal antibody. Homologous CDRs should be understood to include one set of CDRs from an antibody in which the primary sequence of each CDR is at least 50% identical to the antibody and the binding site formed by these CDRs binds to the same epitope as the monoclonal antibody.

The term "chimeric antibody" as used herein refers to antibodies in which the heavy and/or light chains are fusion proteins. Typically the constant domain of the chains is from one particular species and/or class, and the variable domains are from a different species and/or class. Also included is any antibody in which either or both of the heavy or light chains are composed of combinations of sequences mimicking the sequences in antibodies of different sources, whether these sources be differing classes, or different species of origin, and whether or not the fusion point is at the variable/constant boundary. Thus, it is possible to produce antibodies in which neither the constant nor the variable region mimic known antibody sequences. It then becomes possible, for example, to construct antibodies whose variable region has a higher specific affinity for a particular antigen, or whose constant region can elicit enhanced complement fixation, or to make other improvements in properties possessed by a particular constant region. Examples of chimeric antibodies are discussed in U.S. Patent No. 4,816,397 and U.S. Patent No. 4,816,567, the disclosures of which are both herein incorporated by reference.

The term "humanized antibody" as used herein refers to antibodies in which the primary sequence has been altered to make the antibodies less immunogenic in humans. Typically, humanized antibodies derive at least a portion of the framework regions of an immunoglobulin from human immunoglobulin sequences. Humanized antibodies are one type of chimeric antibodies.

Humanization of an antibody can be accomplished by a process known in the art as CDR grafting and described, for example, in U.S. Patent No. 5,225,539, the disclosure of which is herein incorporated by reference. In CDR grafting, the CDRs of a monoclonal antibody, typically a murine monoclonal antibody, which possesses the desired antigen binding specificity are used to replace the CDRs in an otherwise human immunoglobulin. Humanization can also be accomplished by a process known in the art as veneering and described, for example, in WO 92/22653 and EP 0 519 596, the disclosures of which are herein incorporated by reference. Veneering involves substituting amino acids in the framework portions of the variable region of a murine or other non-human monoclonal antibody to make the non-human antibody less immunogenic in a human, while still retaining the desired binding specificity of the non-human antibody for antigen recognized by the non-human CDR region sequences.

A humanized antibody may comprise a substantially complete antibody molecule including all or at least two variable domains capable of recognizing and binding the antigens of interest in which all or substantially all of the CDR regions correspond to those of a non-human immunoglobulin and at least some portion of the FR regions are those of a human immunoglobin sequence. A humanized antibody may also comprise at least a portion of an immunoglobin constant region (Fc), typically that of a human immunoglobulin.

The FR and CDR regions of the humanized antibody need not correspond precisely to the parental sequences. For example, the import CDR or the FR may be mutagenized by substitution, insertion or deletion of at least one residue so that the CDR or FR residue at the site does not correspond exactly to either the FR or the import CDR sequence. Such mutations, however, will not be extensive. Usually, at least 50% of the primary sequence of any particular import CDR will be identical to the sequence in the parent antibody.

The term "altered antibodies" as used herein refers to antibodies in which the naturally occurring amino acid sequence in a vertebrate antibody has been varied. Utilizing recombinant DNA techniques, antibodies can be redesigned to obtain desired characteristics. The possible variations are many, and range from the changing of one or more amino acids to the complete redesign of a region, for example, the constant region. Changes in the constant region, in general, are made to attain desired cellular process characteristics, for example changes in complement fixation where the Fc portions of antibody aggregate to activate the complement system, interaction with membranes where the Fc portion of the antibody binds an Fc cell surface receptor to activate release of the lytic components of the effector cell, and other effector functions. Changes in the variable region of the antibody may be made to alter the antigen binding characteristics, as when the antibody is made to bind antigen with greater affinity. The antibody may also be engineered to aid the specific delivery of a molecule or substance to a specific cell or tissue site. The desired alterations may be made by known techniques in molecular biology including recombinant DNA techniques, and site directed mutagenesis.

A single chain antibody or sFv refers to an antibody prepared by manufacturing the binding regions of the heavy and light chains of an antibody capable of binding the desired antigen and a linking moiety which permits preservation of the binding function. Determination and construction of single chain antibodies are described for example in U.S. Patent No. 4,946,778, the disclosure of which is herein incorporated by reference. An sFv includes at least two polypeptide domains connected by a polypeptide linker spanning the distance between the carboxy-terminal end of one domain and the amino-terminal end of the other domain. The amino acid sequence of each of the polypeptide domains including a set of CDRs interposed between a set of FRs, with the CDRs conferring immunological binding to an antigen of interest. The sFv will have a binding site derived from an antibody, typically a monoclonal antibody. As described in co-pending U.S. Serial Nos. 07/831,967 and 08/133,804, the disclosures of which are herein incorporated by reference, the polypeptide chains of an sFv can bind different epitopes on a preselected antigen, or different epitopes on two different preselected antigens.

Molecules comprising antibody fragments may also be employed in the method of the invention, provided that the antibody fragments retain the antigen binding function of the parent antibody. Fragments of antibodies such as F_{ab}, F_{ab'2}, and Fv and other antibody fragments are within the scope of the definition are useful in the method of the invention. Also included in this definition of antibody fragments are single-chain antibodies, as described herein above.

The term "antibody fragment" as used herein also refers to a polypeptide or group of polypeptides composed of at least one antibody combining site sufficient for binding to the epitope of an antigen. Such an antibody fragment has the three-dimensional binding space including an internal surface shape and charge distribution complementary to the features of an epitope of an antigen, which allows a binding of the antibody with the antigen. The term antibody fragments also includes fragments such as F_{ab}, F_{ab'2}, Fv, and other antibody fragments that retain the antigen binding function of the parent monoclonal antibody are also considered embodied by the invention. The F_{ab} region refers to those portions of the heavy and light chains which are roughly equivalent, or analogous, to the sequences which comprise the branch portion of the heavy and light chains, and which have been shown to exhibit immunological binding to a specified antigen, but which lack the effector Fc portion. F_{ab} includes aggregates of one heavy and one light chain commonly known as F_{ab} or F_{ab'}, as well as tetramers containing the two heavy and two light chains (referred to as F_{ab2}), which are capable of selectively reacting with a designated antigen or antigen family. F_{ab} antibodies may be divided into subsets analogous to those described above, i.e., "hybrid F_{ab}", "chimeric F_{ab}", and "altered F_{ab}". Methods of producing F_{ab} fragments of antibodies are known within the art and include, for example, proteolysis, and synthesis by recombinant DNA techniques.

The term "monoclonal antibody" as used herein refers to an antibody of uniform light and heavy chain composition that may be produced by a single hybridoma, or by recombinant technology as described in Kohler and Milstein, *Nature*, (1975) 256:495-497. Monoclonal antibodies recognize a single epitope, and refer to an antibody composition having a homogeneous antibody population. The term monoclonal antibody is not limited to a particular species or source of the antibody, nor is it intended to be limited by the manner in which it is made. References throughout this description to the use of monoclonal antibodies are intended to include the use of natural or native antibodies as well as humanized and chimeric antibodies. Fragments of antibodies may be derived from, or generated from, the parent monoclonal antibodies. Recombinant forms of these antibodies or fragments may be produced in any expression system conventional in the art, including, for example, prokaryotic, as in bacteria, or eukaryotic, as in yeast, insect or mammalian cells.

Identity or homology with respect to a specified amino acid sequence present in a molecule useful in the method of the invention refers to the percentage of amino acid residues in a candidate sequence that are identical with the specified residues, after aligning the sequences and introducing gaps, if necessary, to achieve the maximum percent homology. None of N-terminal, C-terminal or internal extensions, deletions, or insertions into the specified sequence shall be construed as affecting homology. All sequence alignments called for in this invention are such maximal homology alignments. Alignments may be conducted by hand or with the aid of a computer software program specific for the task. Non-homologous import antibody residues are those residues which are not identical to the amino acid residue at the analogous or corresponding location in a consensus sequence, after the import and consensus sequences are aligned.

To produce antibodies and bispecific antibodies useful for the method of this invention one or more of the following techniques may be used. Hybrid hybridomas (also called tetradomas or Quadromas®) can be employed to produce bispecific antibodies in various media using various techniques known in the art. The production of these hybrid hybridomas and bispecific antibodies involves production of the parental hybridomas, fusion of the parental hybridomas, selection of the hybrid cells, selection of the hybrid hybridomas secreting the desired bispecific antibodies, and purification of the bispecific antibodies. Methods of production of bispecific antibodies are described in WO 92/08802, and U.S. Patent Nos. 4,474,893, and 4,714,681, the disclosures of which are all herein incorporated by reference. The parent cell lines can be fused using standard cell fusion materials and methods. Fusion can be effected using a variety offusogens, including polyethylene glycol. The technique employed is described by Kohler and Milstein, *Nature* (1975) *256*:495-497. Hybrids can be isolated from the mixture of fused cells using a fluorescence-activated cell sorter. Standard techniques are used to set the cell sort windows that are used to select the hybrid cells, and are described by Karawajew et al, *J. Immunol. Methods* (1987) *96*:265-270. After the active cells have been identified, they may be diluted out into a multi-well plate at a concentration of approximately one cell per well with the appropriate growth medium to achieve clonal growth of cell colonies. Detection and selection of the antibody secreting hybrid cells, preferably hybridomas, triomas, and hybrid hybridomas, producing the highest antibody levels may be accomplished as described, for example, in U.S. Patent No. 5,169,774 and WO 90/03186. After the antibody-producing cell has been cultured in the well for a sufficient time, under the appropriate conditions, and has formed a clonal colony, antibody production may be measured.

After the appropriate cells have been selected, they may be cultured and fermented under a variety of conditions that favor antibody production. See, for example, U.S. Patent No. 5,096,816; WO 89/01027; WO 89/01028; WO 89/01029; WO 90/03429; WO 90/03430; Velez *et al, J. Imm. Meth*. (1986) 86:45-52; Reuveny *et al, J. Imm. Meth.* (1986) *86*:53-59; and Reuveny *et al, J. Imm. Meth.* (1986) *86*:61-69.

The term "3G8" as used herein refers to a monoclonal antibody produced by a hybridoma 3G8. 3G8 recognizes and binds to FcγRIII which binds the Fc fragment of aggregated IgG. Hybridoma 3G8 was initially disclosed in Unkeless *et al, J. Exp. Med*. (1979) 150:580-596.

The term "self antigen" as used herein refers to antigens encoded by the genome of and present in a patient. Generally, the immune system does not respond to self antigens and develops a condition of tolerance to them. Immunological tolerance reflects an adaptive response of the immune system. T cell clones recognizing self antigens are believed to deleted during development. It is believed that there are at least three other reasons why self antigens, particularly those present on cancer cells, may not be recognized by the immune system. In some cases, the structure of self antigens may make them difficult for macrophages to phagocytose and process these antigens. In other cases, the cells on which a self antigen is present may lose its ability to present antigen. Finally, some cancer cells are believed to secrete immunosuppressive cytokines thereby preventing the recognition of the self antigens present on the surface of the cancer cell.

The term "cancer antigens" as used herein refers to antigens expressed on the surface of cancerous cells and which may serve as a marker for the cancerous condition of the cell. Some of these antigens have been used to track the progress of the disease, and are either unique to cancerous cells, or are present in larger numbers on cancer cells. Cancer antigens thus serve as molecular markers, differentiating cancer cells from normal cells. Cancer antigens are useful in the method of the present invention in the targeted induction of an immune response by administering a bispecific antibody specific for a cytotoxic cell antigen and a cancer antigen expressed by the cancer cells.

Cancer antigens include, without limitation, *c-erb*B-2 (erbB-2), and other antigens structurally and functionally related to growth factor receptors. The *erb*B-2 antigen is related to the epidermal growth factor receptor (EGF receptor), and has also been referred to in the art as c-*neu* or HER-2. The erbB-2 protein product, p185, binds a distinct 30 kD factor secreted by breast cancer cells (gp30). The hallmark of antigens in this family (those related to the EGF receptor) is the presence of two regions rich in cysteine residues in the external domain of their protein products. Antibodies to cancer antigen c-*erb*B-2 can be obtained, for example from the hybridoma cell line 520C9, HB 8696. Expression of protooncogene c-*erb*B-2 in fresh human tumors and tumor cell lines has been studied extensively as described in Kraus *et al*, *EMBO J*. (1987) 6:605-610; Hynes *et al, J. Cell Biochem.* (1989) 39:167-173; Fukumoto *et al, PNAS* (1988) 85:6846-50; Gullick *et al, Int. J. Cancer* (1987) 40:246-254; Natali *et al, J. Int. Cancer* (1990) 45:457-461; Zajchowski *et al, Cancer Res.* (1988) 48:7041-7047. Thirty to 40% of human breast, ovarian, and colon tumors have been found to express erbB-2. Correlation between amplification of erbB-2 and overexpression of erbB-2 in breast and ovarian tumors and poor prognosis for the patients have been investigated by various groups. Other human tumors and cell lines that have been associated with overexpression of erbB-2 include neuroblastoma, lung cancer, thyroid cancer, pancreatic cancer, prostate cancer, renal cancer and cancers of the digestive tract. Localization to or growth inhibition of erbB-2-positive tumors with monoclonal antibodies has been reported in an *in vitro* model as described in Hseih-Ma *et al, Cancer Res.* (1992) 52:6832-39.

Another class of cancer antigen capable of use in the method of the invention are oncofetal proteins of nonenzymatic function. The antigens are found in a variety of neoplasms, and are often referred to as tumor-associated antigens. Carcinoembryonic antigen (CEA), and a-fetoprotein (AFP) are two examples. AFP levels rise in patients with hepatocellular carcinoma: 69% of patients with liver cancer express high levels of AFP in their serum. CEA is a serum glycoprotein of 200 kD found in adenocarcinoma of colon, as well as cancers of the lung and genitourinary tract. It has been hypothesized that overproduction of CEA in colonic tumors may disrupt normally operating intercellular adhesion forces, resulting in more cell movement and less differentiation, such as occurs in the early stages of tumorigenesis.

Yet another class of cancer antigens are those antigens unique to a particular tumor, referred to herein as "tumor specific antigens". As described in Udono *et al*, (1993) J. Exp. Med. 176:1391-96, the disclosure of which is herein incorporated by reference, vaccination of mice with heat shock protein 70 (hsp70) preparations from a Meth A sarcoma rendered the mice immune to a subsequent substantial challenge with Meth A sarcoma. Vaccination with hsp70 from normal tissue did not protect from challenge with Meth A sarcoma, nor did vaccination with hsp70 prepared from other tumor sources. Udono *et al* believe that antigenic peptides were chaperoned by hsp70 and that these antigens were tumor specific. Normal human cells may contain silent mutations in many types of proteins including housekeeping proteins such as actin, in metabolic proteins such as enzymes and in cell surface proteins. These mutated proteins typically avoid detection by the immune system, with a corresponding immune response, because they are unique to one particular cell. In a cancer setting, where one immortal cell gives rise to millions of clonal derivatives in one or more tumors, the silent mutation now may provide a mechanism for the immune system to recognize and respond to the cancer cell. It is believed that the method of the invention in which bispecific molecules capable of recognizing and binding a cancer antigen such as erbB-2, can also induce an immune response to this class of tumor specific antigens. Without being limited to one particular explanation, it is believed that targeting cancer cells for phagocytosis by macrophages, using a bispecific molecule such as 2B1, results in processing of tumor cell peptides with presentation of these peptide including tumor specific antigens on proteins, such as hsp70 or hsp90.

Antibodies to CEA have been developed as described in U.K. 2 276 169, the disclosure of which is herein incorporated by reference. The antibodies to CEA are humanized antibody molecules derived from murine monoclonal antibodies to CEA. These antibodies retain specificity for CEA with an antigen binding site of at least one CDR of the variable domain of the murine parent antibody A5B7. The sequence of the variable region of A5B7 is also disclosed in U.K. 2 276 169.

Antibodies to other cancer antigens which are useful in the method of the invention include, antibody 42H8 to a 145 kD cancer antigen, 452F2, 741F8, 520C9, 759E3, 113F1 and 454C11 all to erbB-2 cancer antigen, 387H9 to a 40 kD cancer antigen, 113F1 to an epitope found on 40 kD, 60 kD, and 100 kD cancer antigens of various cancers, 317G5, 34F2, 650E2 and 35E6 all to a 42 kD cancer antigen, 266B2, 106A10 and 260F9, all to a 55 kD cancer antigen, 33F8 to a 66 kD cancer antigen, 9C6 to a 75 kD cancer antigen, 35E10 to an 80 kD cancer antigen, 140A7 and 36H3 to a glycolipid cancer antigen, 788G6, 200F9, 697B3, 120H7, 203E2, 254H9, 245E7, and 2G3 all to a high molecular weight (HMW) mucin cancer antigen, 369F10 to a different HMW mucin cancer antigen (designated HMW mucin II), 15D3 to p-glycoprotein cancer antigen, 421E8, 310B7, 32A1, 219F3, 42E7, and 388D4 to various cancer antigens from cancers including colon cancer and other cancers as well.

Both monoclonal antibodies 140A7 and 36H3 bind to glycolipids extracted by chloroform/methanol extraction from MCF-7 breast cancer cells. 3GH3 binds preferentially to neutral glycolipids, and 140A7 binds preferentially to a monosialo ganglioside fraction. The monoclonal antibodies are further distinguished in that binding of 3GH3 is insensitive to oxidation of the target glycolipids with sodium periodate, while binding of 140A7 is diminished by periodate treatment of the glycolipids.

15D3 binds to p-glycoprotein (or mdr), the expression of which is associated with multi-drug resistance often observed in certain cancer cells resistant to chemotherapy. P-glycoprotein RNA or protein has been detected in several cancers, and the expression of p-glycoprotein is associated with progression of cancer, as described in U.S. Patent Nos. 4,912,039 and 5,206,352, the disclosures of which are both herein incorporated by reference.

Other cancer antigens to which an immune response can be generated using the method of the invention, include the following cancer antigens: CA 195 tumor-associated antigen-like antigen, isolated from human amniotic fluid, and useful for measuring CA 195 in human serum as described in U.S. Patent No. 5,324,822, herein incorporated by reference; squamous cell carcinoma-like antigens from female urine as described in U.S. Patent No. 5,306,811, herein incorporated by reference; and a glycoprotein antigen found on the surface of normal and benign breast epithelial cell membranes and on breast cancer cells as described in U.S. Patent No. 4,960,716, herein incorporated by reference.

Antibodies to other cancer antigens which are useful in the method of the invention include, antibody 42H8 to a 145 kD cancer antigen, 35E6 to a 42 kD cancer antigen, and 36H3 to a glycolipid cancer antigen. Monoclonal antibody to 36H3 bind to glycolipids extracted by chloroform/methanol extraction from MCF-7 breast cancer cells.

Other cancer antigens to which an immune response can be generated using the method of the invention, include the following cancer antigens: CA 195 tumor-associated antigen-like antigen, isolated from human amniotic fluid, and useful for measuring CA 195 in human serum as described in U.S. Patent No. 5,324,822, herein incorporated by reference; squamous cell carcinoma-like antigens from female urine as described in U.S. Patent No. 5,306,811, herein incorporated by reference; and a glycoprotein antigen found on the surface of normal and benign breast epithelial cell membranes and on breast cancer cells as described in U.S. Patent No. 4,960,716, herein incorporated by reference.

The term "cancer ganglioside" as used herein refers to a specific glycosphingolipid component of a tumor cell located in the plasma membrane. Gangliosides contain a particular type of acidic carbohydrate known as sialic acid. Many tumor cells are characterized by the particular ganglioside in their plasma membrane. A specific monoclonal antibody to a cancer gangliosides are, for example, described in U.S. Patent No. 5,389,530, herein incorporated by reference.

The term "2B1" as used herein refers to a hybrid hybridoma capable of producing a bispecific monoclonal antibody useful in the method of the invention. 2B1 (CRL 10197) is a product resulting from the fusion of the parent hybridomas 520C9 (HB 8696) and 3G8. Administration of 281 to a patient promotes erbB-2 (+) tumor lysis by NK cells and macrophages expressing the FcγRIIIA isoform of CD16 (hereinafter referred to as "FcγRIII").

As an example, the following describes the process of making the bispecific antibody 2B1 (CRL 10197). Hybridoma 520C9 (HB 8696) recognizing the protooncogene product c-erbB-2 and hybridoma 3G8 recognizing the human FcγRIII were stained with vital fluorescent dyes and fused with PEG to form hybrid hybridomas; these hybrid hybridomas were isolated by dual fluorescence on a cell sorter. Cultures whose supernatants promoted lysis of SK-Br-3 breast cancer cells by human total mononuclear cells were chosen for subcloning. The immunoglobin species produced by clone 2B1 could be resolved on anion exchange chromatography into parental antibody 520C9, a middle peak containing bispecific antibody, and parental antibody 3G8. The bispecific peak further resolved into major and minor peaks on cation exchange chromatography. The major peak appeared bispecific on the basis of SDS PAGE, isoelectric focusing, immunofluorescent binding to both tumor and effector cells, and ability to promote targeted cytolysis. The minor peak also appeared bispecific by gel criteria, but lacked tumor cell binding and cytolytic targeting ability. It may be that the minor species of bispecific antibody is more heavily glycosylated at the hinge region in a manner that sterically interferes with activity of the 520C9 but not the 3G8 binding site. Purified 2B1 bispecific antibody promoted lysis of c-erbB-2 positive breast, ovarian and lung cancer cells by human NK cells (also called large granular lymphocytes, or LGL) and macrophages. It should be understood that the term "2B1" as used herein may also refer to the bispecific antibody produced by the 2B1 hybrid hybridoma.

Bispecific antibody from 2B1 has been shown to promote specific lysis of erbB-2 positive tumor cells in vitro as described in Hsieh-Ma et al, Cancer Research (1992) 52:6832-39. In short-term ⁵¹Cr release assays with human mononuclear cells as effectors and SK-Br-3 human breast cancer cells as targets, neither parental antibody of 2B1 mediated specific lysis, but bispecific antibody 2B1 at a concentration of 2 ng/ml caused half-maximal lysis at an effector/target ration of 20:1, and 2 ng/ml 2B1 caused half maximal lysis at an effector/target ratio of 40:1. The cytotoxic targeting activity of the 2B1 F_{ab'2} fragment was the same as that of the whole bispecific antibody. The activity of whole 2B1 was not reduced when assays were performed in 100% autologous human serum, indicating that 2B1 binds effector cells through the CD16-binding site derived from parental antibody 3G8 rather than through its Fc portion. 2B1 was also able to mediate targeted cytolysis using whole human blood as a source of effector cells or using effector or target cells derived from ovarian cancer patient.

The term "1A7" (HB 10501) as used herein refers to a hybrid hybridoma capable of producing bispecific antibodies useful in the method of the invention. Bispecific antibodies produced by 1A7 are capable of recognizing and binding FcγRIII and anti-p-glycoprotein (also called multi-drug resistance antigen or mdr). The 1A7 hybrid hybridoma was formed from the fusion of two parental hybridomas 15D3 (HB 11342) and 3G8.

The term "viral antigens" as used herein refers to antigens present on the capsids or envelopes of viruses and to antigens expressed on the surfaces of virally infected cells. Viral antigens include, without limitation, antigens produced by human immune virus (HIV), hepatitis A virus (HAV), hepatitis B virus (HBV), hepatitis C virus (HCV), human papilloma virus (HPV), herpes simplex virus (HSV), cytomegalovirus (CMV), Epstein-Barr virus (EBV), and Influenza virus.

A specific viral antibody useful in the method of the invention is, for example, the anti-HIV monoclonal antibody to the V3-PND region of glycoprotein antigen gp120, as described in WO 94/20632, the disclosure of which is herein incorporated by reference.

Two specific anti-HAV monoclonal antibodies useful in the method of the invention are, for example, K3-4C8 and B5-B3, each which recognize neutralizing epitopes of the virus, as described in Delem *et al. Vaccine* (1993) 11 (4)479-84, the disclosure of which is herein incorporated by reference.

Specific anti-HBV monoclonal antibodies useful in the method of the invention are, for example, those described in Budkowska *et al. J. of Virology* (1995) 69(2):840-8, the disclosure of which is herein incorporated by reference, to the important and potentially neutralizing epitopes located in the pre-S1, pre-S2, or S region-encoded domains of HBV proteins.

A specific anti-HCV monoclonal antibody useful in the method of the invention is, for example, a monoclonal antibody to the HCV E2 antigen (gp68-72) as described in Selby *et al. J. of Gen. Virol.* (1993) 74: 1103-1113, the disclosure of which is herein incorporated by reference.

A specific anti-HPV monoclonal antibody useful in the method of the invention is, for example, H11.A3 to the HPV-II antigen as described in Christensen *et al. J. of Virol*. (1990) 64 (11): 5678-5681, the disclosure of which is herein incorporated by reference.

A specific anti-HSV monoclonal antibody useful for the method of the invention is, for example, available from Bios Pacific, 4240 Hollis Street, #290 Emeryville, CA 94608, (510-652-6155). As described in their specification, the disclosure of which is herein incorporated by reference, clone A19080023A is an anti-HSV-I antibody to the envelope glycoprotein gB of HSV-I.

A specific anti-CMV monoclonal antibody useful for the method of the invention is, for example, available from Bios Pacific, 4240 Hollis Street, #290 Emeryville, CA 94608, (510-652-6155). As described in their specification, the disclosure of which is herein incorporated by reference, clone A28020069P is an anti-CMV antibody to an immediate early nonstructural antigen (68-72 kD) and does not cross react with EBV, ADV, VZV, or HSV. Another specific anti-CMV monoclonal antibody useful for the method of the invention is, for example, the antibody to the major CMV envelope glycoprotein, gB (gp UL 55), as described in Schleiss *et al*. *Virology* (1994) 202:173-185, the disclosure of which is herein incorporated by reference.

A specific anti-EBV virus monoclonal antibody useful in the method of the invention is VCA-IgG (antibody to viral capsid antigen), as described in Andersson *et al. J. Med. Virol*. (1994) 43:238-244, the disclosure of which is herein incorporated by reference, and the latent membrane protein and Epstein-Barr virus nuclear antigen-2 antigens both as described in Pinkus *et al. Modern Pathol.* (1994) 7(4):454-61, which is herein incorporated by reference.

Specific anti-Flu virus monoclonal antibodies useful in the method of the invention are, for example, available from Bios Pacific, 4240 Hollis Street, #290 Emeryville, CA 94608, (510-652-6155). As described in their specification, the disclosure of which is herein incorporated by reference, clone A60010044P is an anti-Influeza A antibody to viral subtypes H1N1, H2N2, and H3N2, and clone A60020044A is an anti-Influenza B antibody to Influenza B flu virus antigen.

The term "fungal antigens" as used herein refers to antigens from any of the saprophytic and parasitic lower plants belonging to the major group Fungi. Fungi as a division of lower plants includes saprophytic and parasitic plants lacking chlorophyll and comprising the classes Phycomycetes, Ascomycetes, Basidiomycetes, Fungi Imperfecti, and also Myxomycetes and Schizomycetes. These classes include molds, rusts, mildews, smuts, and mushrooms. Fungal antigens will be found on those fungi capable of pathogenesis in mammals.

Specific fungal monoclonal antibodies useful for the method of the invention are, for example, antibodies to non-enhancing protective epitopes on serotype A, B, C, and D strains of C. neoformans as described in WO 93/08271 , the disclosure of which is herein incorporated by reference.

The term "parasitic antigens" as used herein refers to antigens from organisms which live in or on another organism in an intimate association in which a parasite obtains benefits from a host which is usually injured. An example of a disease caused by a parasite is malaria. Malaria is an acute or chronic disease caused by the presence of sporozoan parasites of the genus Plasmodium, transmitted from infected human to infected human by the bite of infected anopheline mosquitoes. Once transferred to a host, the protozoan multiplies asexual by schizogony, and causes destruction of the red blood cells.

A specific parasitic monoclonal antibody useful for the method of the invention is, for example, monoclonal antibody to PfEMP3 Malaria antigen as described in WO 94/03604, the disclosure of which is herein incorporated by reference.

The term "toxins" as used herein refers to refers to other poisonous substances which may or may not be present in a patient and to which an immune response can be generated. The term "toxins" as used herein includes, for example, the colloidal proteinaceous poisonous substances that are a specific product of the metabolic activities of a living organism and are usually unstable and toxic when introduced into tissues, typically inducing antibody formation. A specific monoclonal antibody to toxin useful for the method of the invention is, for example, antibodies to the heavy chain fragment of tetanus toxin as described in WO 94/00487, the disclosure of which is herein incorporated by reference.

The phrase "antigen not present in the patient upon first administration" as used herein refers to antigen not present or not known to be present upon prophylactic administration of a bispecific antibody useful in the method of the invention. Such an administration might occur, for example, in patients with high risk for infection by a viral or bacterial pathogen or at risk for exposure to toxins. It will be appreciated that methods for detecting antigen in a patient may fail to detect the antigen despite the presence in a biological sample from the patient. Therefore, antigen not present in the patient should be understood to include circumstances in which an antigen, though present in the patient, has not yet been detected.

The process of inducing an immune response with the administration of a bispecific antibody to FcγRIII and a tumor antigen may involve 2B1 inducing macrophages which are in the tumor, in contact with the tumor cells, to phagocytose and lyse these tumor cells. Phagocytosis and lysis of the tumor cells would result in presentation of c-erbB2 antigen as well as presentation of other tumor specific antigens released by or present on the surface of the lysed tumor cell. If the tumor had previously escaped rejection because of various immune defects or defenses, this mode of antigen presentation via 2B1 may bypass such defenses and lead to responses against a variety of tumor epitopes.

The present invention will now be illustrated by reference to the following examples, which set forth certain embodiments. However, it should be noted that these embodiments are illustrative and are not to be construed as restricting the invention in any way.

### Example 1

Evaluation of erbB-2 antibody in a 2B1 bispecific antibody clinical trial was conducted. Eleven patients with a variety of erbB-2 overexpressing primary tumors were treated on phase I dose escalation trial of 2B1 bispecific monoclonal antibody. The doses were given IV on days 1, 4, 5, 6, 7, and 8. Response is indicated as progressive disease (P.D.), stable disease (S.D.) or partial response (P.R.) in Table I. Pre-treatment circulating serum erbB-2 ECD levels (serum ECD) were determined by EIA. Pre- and post-treatment antibody responses to erbB-2 antibodies were detected by an erbB-2 antibody ELISA. Patient anti-mouse antibody responses (anti-mouse antibodies) were determined by an ELISA technique using mouse antibody as the plate-coating antigen. Anti-idiotypic antibodies for the erbB-2 binding arm of the 2B1 bispecific antibody (anti-2B1 idiotypic antibody) were determined by a blocking ELISA using the 2B1 parent antibody and purified erbB-2 protein to block human serum binding.

More specifically, patients with erbB-2 (+) tumors were treated with 1 hr. i.v. 2B1 infusions on days 1, 4, 5, 6, 7 and 8 at 1 mg/m² (n=3), 2.5 mg/m² (n=6), or 5.0 mg/m² (n=6) per dose, in a dose-escalating Phase I clinical trial. Humoral immune responses were evaluated in these patients. Significant HAMA (human anti-mouse antibody) responses were detected against whole 2B1 IgG and its associated antigen-binding domains in a range from 70 to >50,000 ng/ml, using primate anti-mouse IgG reactivity as a standard as shown in the anti-mouse antibody post treatment column of Table 1. HAMA was consistently elevated from days 11 through 222 following the start of treatment. Peak anti-idiotypic antibody responses were demonstrated to the F_{ab'2} parental antibodies 520C9 (anti-erbB-2) and 3G8 (anti-CD16) between days 25 and 38 with a downward trend thereafter.

### Example 2

To determine whether the 2B1 bispecific antibody could be used to elicit an immune response to erbB-2, serial serum samples from patients in the clinical trial described in Example 1 were examined. Serum samples from the first 11 patients treated with the 2B1 antibody in a dose escalation trial were examined for the generation of an immune response to erbB-2 protein. Patients with a variety of different histologic types of erbB-2 overexpressing primary tumors were included. Baseline and day 11 serum samples were available on all patients, and several later time points were collected on the majority. None of the 11 patients had detectable erbB-2 antibodies prior to therapy. These results are shown in the column labelled anti-H2N antibody of Table 1.

A direct ELISA was used in assaying erbB-2 antibody immunity in 2B1-treated patients. Purified erbB-2 protein was prepared via an immunoaffinity column erbB-2 purification scheme using a monoclonal antibody directed against the erbB-2 molecule covalently linked to a gel support column. Lysates of the SKBR3 cell line were used as a source of erbB-2 protein to bind the column, and after extensive washing to remove extraneous non-specific proteins from the column, the erbB-2 molecule was isolated using acid elution. After dialysis and protein quantitation, the protein was used to coat Immulon® plates. Blocking of any remaining protein binding sites was accomplished with PBS/1% BSA. Human serum collected from 2B1 patients was then added to the plate and titrated in two-fold dilutions beginning at 1:25. The detection reagent, sheep anti-human immunoglobulin conjugated in horseradish peroxidase, was then added to the system. Color development was initiated with the chromogen TMB and stopped with acid when a positive control well reached predetermined optical density (O.D.) reading. The O.D. of the entire plate was then measured at 450 nanometers. Negative and positive control serum samples were also included on each plate. Patients with erbB-2 -specific antibody reading two standard deviations above the mean of a normal control population were considered to have positive antibody responses for the erbB-2 protein.

All serum erbB-2 ECD protein testing was done using an enzyme immunoassay (EIA) developed by Triton Diagnostics, although other similar test kits may also be used for this assay. This assay system involves two enzyme-conjugated antibodies specific for the extracellular domain of the erbB-2 protein. The O.D. readings of the samples are compared to a standard curve generated using known quantities of ECD protein. The results are expressed in ECD units/ml. The cutoff value for a positive result has been determined as two standard deviations from a mean reading in a normal control population.

The presence of erbB-2 antibody immunity following treatment was confirmed by showing that serum antibodies could immunoprecipitate the 185 kD erbB-2 molecule post-therapy, but not pre-therapy. Samples used for immunoprecipitation were from time points well after all mouse 2B1 antibody cleared the patients' circulation, in order to preempt false positive or negative results due to mouse anti-erbB-2 antibody.

Studies using a Western blot assay for epitope mapping data showed that the antibodies generated in these patients recognized sites on both the intracellular domain (hereinafter "ICD") and extracellular domain (hereinafter "ECD" of the erbB-2 molecule. Since anti-idiotypic antibodies were detected to the erbB-2 binding region of the 2B1 bispecific antibody, which recognizes the extracellular region of the receptor, it is possible that human antibodies developed against the ECD arose though an idiotype/anti-idiotype network. For antibodies to be generated against the ICD, however, some other mechanism of immune processing and recognition is implicated. A likely explanation would be that the immune effector cells targeted by the CD16 receptor arm of the 2B1 antibody has to be involved in this process. The induced erbB-2 antibodies were for the most part IgG in isotype at day 30 and thereafter, which would imply T helper cell involvement in antibody class switching from IgM in a primary immune response.

### Example 3

This example demonstrates the induction of an immune response by administration of a bispecific antibody such as 2B1. Administration of 2B1 can result in an immune response including the presentation of the tumor antigen erbB-2, and can also result in activating macrophages to lyse and phagocytose tumor cells resulting in presentation of many antigens present in the tumor cells.

Monoclonal antibodies to a number of tumor antigens other than c-erbB-2 are used to set up competitive immunoassays to detect whether patients have antibodies to those antigens before and after treatment with 2B1. For example, microtiter wells are coated with a nonionic detergent extract of a tumor cell line such as MCF-7 or SK-BR-3 to allow adsorption of a variety of tumor antigens to the plastic surface. The cell line used is chosen to provide tumor antigens recognized by available antibody probes. Excess tumor cell extract is washed away, and the surface is blocked with a solution containing one or more suitable agents such as bovine serum albumin, nonfat dry milk proteins or Tween 20 in order to minimize subsequent nonspecific adsorption of proteins. After washing away the blocking solution, dilutions of plasma or serum samples obtained from patients before and at various time points after bispecific antibody treatment are added to the wells and incubated to allow specific binding of patient antibodies that recognize tumor antigens adsorbed on the wells. The wells are then washed to remove unbound antibodies, including HAMA. A solution containing a probe (e.g., a monoclonal antibody conjugated to HRP) specific for a tumor antigen of interest is then added to the wells and incubated to allow binding of the probe to the antigen. The probe solution may contain mouse serum or one or more irrelevant mouse antibodies in order to prevent binding of the probe antibody to any HAMA that has been nonspecifically adsorbed to the wells. If the patient's serum or plasma contained antibody specific for the cancer antigen epitope recognized by the probe, binding of the probe will be diminished or eliminated. After incubation, excess probe is washed out of the wells, and bound probe is quantitated by adding a solution containing a chromogenic substrate, incubating to allow color development, and reading the plate on an ELISA plate reader.

Alternatively, microtiter wells may be coated with a tumor cell extract that does not contain the particular tumor antigen recognized by the bispecific antibody used to treat patients. The wells are then blocked and exposed to dilutions of patient serum or plasma as described above. Rather than using a probe specific for a particular cancer antigen, the wells are then incubated with an antibody-HRP probe specific for human immunoglobulin, and probe binding is measured as described above. If more human immunoglobulin binds to the wells after bispecific antibody treatment than prior to treatment, one would conclude that treatment has caused development of antibodies to tumor antigens. Since the tumor cell extract was chosen not to contain the particular tumor antigen recognized by the bispecific antibody, one would further conclude that different tumor antigens are being recognized.

### Example 4

To study bispecific antibody induction of T cell responses, several T cell assays can be constructed. Helper T cell responses can be followed by measuring T cell proliferation 16-20 or IL-2 secretion after exposure to antigen-presenting cells supplied with the antigen of interest as described in Snider and Segal, (1987) J. Immunol. 139:1609-16. Cytotoxic T cell responses are detected by lysis of radiolabelled antigen presenting cells primed with relevant tumor antigens described in Disis *et al*, (1994) Cancer Research 54:1071-1076. The disclosure of the references in this Example 4 are herein incorporated by reference.

### Deposit Information

The ATCC accession numbers for the antibody producing hybridomas disclosed herein are 42H8 (HB 11830) 35E6 (HB 11769) and 36H3 (HB 11768). The American Type Culture Collection is located at 12301 Parklawn Drive, Rockville, MD, USA (ATCC).

These deposits were made under the provisions of the Budapest Treaty on the International Recognition of the Deposit of Microorganisms for the Purpose of Patent Procedure and the Regulations thereunder (Budapest Treaty). This deposit assures maintenance of viable cultures for 30 years from the date of the deposit. The organisms will be made available by ATCC under the terms of the Budapest Treaty, and subject to an agreement between Chiron Corporation and ATCC, which assures permanent and unrestricted availability of the progeny of cultures to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 U.S.C. §122 and the Commissioner's rules pursuant thereto (including 37 C.F.R. §1.12 with reference made to 886 O.G. 638).

The patents, patent applications and publications cited herein are incorporated by reference.

The present invention has been described with reference to specific embodiments. However, this application is intended to cover those changes and substitutions which may be made by those skilled in the art without departing from the spirit and the scope of the appended claims.

## Claims

1. A monoclonal antibody which is obtainable from the hybridoma deposited with the American Type Culture Collection having Accession No. HB 11751.

2. A monoclonal antibody according to claim 1, which is specific to a colon cancer antigen.

3. An antigen bound by monoclonal antibody 42E7 which is produced by the hybridoma deposited with the American Type Culture Collection having Accession No. HB 11751.

4. An antigen according to claim 3, which is a human cancer antigen.

5. An antigen according to claim 4, which is a colon cancer antigen.

6. A monoclonal antibody that binds to a cancer antigen according to claim 3, 4 or 5.

7. Hybridoma deposited with the American Type Culture Collection having Accession No. HB 11751.

8. Use of an antibody according to claim 1, 2 or 6 in the manufacture of a medicament for inducing an immune response to said antigen in a patient.

9. Use of an antibody according to claim 1, 2 or 6 in the manufacture of a medicament for diagnosing or treating cancer in a human subject.

10. Use of an antigen according to claim 3, 4 or 5 in the manufacture of a medicament for inducing an immune response to said antigen in a patient.

11. Use of an antigen according to claim 3, 4 or 5 in the manufacture of a medicament for diagnosing or treating cancer in a human subject.
